# EUROPEAN PATENT APPLICATION

(11) **EP 1 669 097 A2**
(43) Date of publication of application: **14.06.2006**
(21) Application number: 01102571.5
(22) Date of filing: 06.02.2001
(51) Int. Cl.: A61M 5/32, A61M 5/00

(54) **Syringe storage and disposal container**

(71) Applicant: LICENCE MANAGEMENT Pty Limited, Sydney, New South Wales 2000 (AU)
(72) Inventor: Vumbaca, Gino Anthony, Allambie Heights, New South Wales 2100 (AU)
(74) Representative: Staudt, Hans-Peter

(57) **Abstract**

A syringe container (30) has three pockets (36,37,38) each with parallel walls (39,40) having resiliently deformable lugs (41,42) which extend inwardly from each wall. The lugs (41,42) are separated by a distance sufficient to permit passage of the body (43) of a syringe (44) but prevent passage of the flange (45) of a syringe unless the lugs are caused to deform by application of a force to the syringe. The pocket is arranged so that a sterile syringe can be contained completely within the container, the sterile syringe can then be removed and used and subsequently returned to the container to be retained by pushing the flange of the syringe beyond the lugs.

## Description

The present invention relates to a syringe container for retaining soiled syringes such that syringes retained within the container cannot be removed from the container without the application of excessive force to or destruction of the container.

The devastating effect upon the population at large caused by the HIV virus is widely acknowledged and various attempts ranging from education to incarceration of carriers have been made to stem the spread of the virus. Contact with contaminated blood is an acknowledged vehicle for transfer of the virus and one specific form of such transfer is the sharing of syringes by intravenous drug users. The present invention seeks to reduce the likelihood of the transfer of the HIV virus by providing a tamper-proof container for soiled syringes thereby reducing the number of soiled syringes that are inappropriately disposed of in the community and to encourage intravenous drug users not to share syringes. Although the invention is described in this context it is to be understood to have wider application and is in fact appropriate for retaining any soiled syringe.

In recognition of the above, the applicant has developed the present invention and it is envisaged that containers filled with sterile syringes will be made available free of charge or for a nominal charge to intravenous drug users through pharmacies or the like. It is then intended that the drug user will use a sterile syringe from the container for each injection and after use return the soiled syringe to be retained within the tamper-proof container. When all sterile syringes have been used and returned to the container, the container is returned to a pharmacy or the like and effectively swapped for a further container containing sterile syringes. Arrangements are then to be put in place for the safe disposal of the containers containing the soiled syringes. Typically this would involve collection of returned containers and their incineration along with other commonly incinerated medical refuse.

It can therefore be seen that if used in the manner envisaged, the present invention should reduce the spread of the HIV virus by reducing the chance of members of the public accidentally contacting a soiled syringe which has been inappropriately disposed of in a public area and should encourage intravenous drug users not to share needles.

Preferred embodiments of the present invention are presently being used in the Australian state of New South Wales and are referred to as "Fitpacks". The use of Fitpacks in other Australian states is planned for the near future.

Prior to the advent of the present invention, the New South Wales State Government had established thirty Needle and Syringe Exchange Programmes (NSEPs) which between them operated from approximately eighty outlets. These outlets varied from a dedicated fixed location such as one service offered by a sexually transmitted diseases clinic to outreach services including needle and syringe distribution from vehicles in public areas.

In addition to the established NSEPs, arrangements have been made with the Pharmacy Guild of Australia to distribute and exchange Fitpacks. There are presently over 400 pharmacies throughout New South Wales dispensing Fitpacks. The Fitpacks are dispensed and exchanged free of charge from NSEPs and exchanged free of charge on an old for new exchange basis from pharmacies. In the absence of a used Fitpack for exchange, new Fitpacks can be purchased from pharmacies for A$3.00. The New South Wales State Government therefore bears the brunt of the costs associated with the programme but this is viewed as being desirable when the object of the programme is taken into consideration.

NSEPs order empty Fitpacks directly from the manufacturer whilst arrangements have been made for the pharmacies to obtain empty Fitpacks from their regular wholesalers. Needles and syringes are obtained independently and the empty Fitpacks are filled with needles and syringes at the various points of distribution by NSEP workers and pharmacists.

Individual arrangements have been made with each NSEP and pharmacy for the safe disposal of used Fitpacks under established clinical waste guidelines. The disposal involves incineration above 1100°C and it is noted that the preferred embodiments of the invention presently used are manufactured from polypropylene which does not create environmental hazards upon incineration.

No quantitative data is available to date for the return rate of used Fitpacks but the qualitative observation has been that the rate of return has exceeded 65% which was the figure prior to the introduction of the Fitpack programme.

In US 3348894, forming the preamble of claim 1, a cabinet for hypodermic syringes is described. The cabinet consists of a series of compartments for sterile syringes and a separate compartment for contaminated syringe parts. The contaminated syringe compartment has an opening which is separate from the apertures to the sterile syringe compartments. Moreover, the contaminated syringe compartment has a separate closure so that the contaminated syringe compartment can remain closed when the sterile syringe compartments are open. Turning now to US 4520926, this document describes a retaining device for contaminated syringes which has deformable lugs to hold the syringes in place.

It is an object of the present invention to provide a syringe container for retaining soiled syringes such that syringes retained within the container cannot be removed from the container without application of excessive force to or destruction of the container.

The present invention provides a container in accordance with the appended claims hereto.

The container has a pocket having two substantially parallel walls extending away from the open end of the shell. Two said lugs are provided extending inwardly from the walls of the pocket and the lugs are at the same level in the pocket. The lugs are resiliently deformable and biased to positions in which they are separated by a distance which is sufficient to permit passage of the body of a syringe. The body of a syringe is to be understood to be the barrel which houses the plunger, the needle and moulding which attaches it to the barrel and the cap which fits over the needle if fitted. The distance between the lugs is however insufficient to permit passage of the flange of a syringe which is to be understood to be that part of a syringe upon which the index finger and third digit generally rest when force is applied to the plunger by the thumb. Upon application of sufficient force, the lugs deform to permit passage of the flange and then return to their original position.

Preferably, the lugs are sufficiently remote from the open end of the shell housing to allow a sterile syringe to be completely within the container when the flange of the sterile syringe rests on the lugs. Accordingly, a sterile syringe can rest upon the lugs, be removed from the container and used, and subsequently returned to the container to be retained following pushing the flange of the syringe beyond the lugs. Each pocket is therefore designed to contain one sterile syringe and subsequently retain one soiled syringe. A container can therefore comprise a plurality of pockets to contain and subsequently retain a corresponding plurality of syringes.

The shell and the retaining means are preferably separately moulded from plastics material and ultra-sonically welded together.

Notwithstanding any other forms that may fall within its scope, a preferred embodiment of the invention will now be described, by way of example only, with reference to the accompanying drawings, in which:
Figure 1 is a plan view of a syringe container not in accordance with the invention showing the lid of the container in the fully opened position,

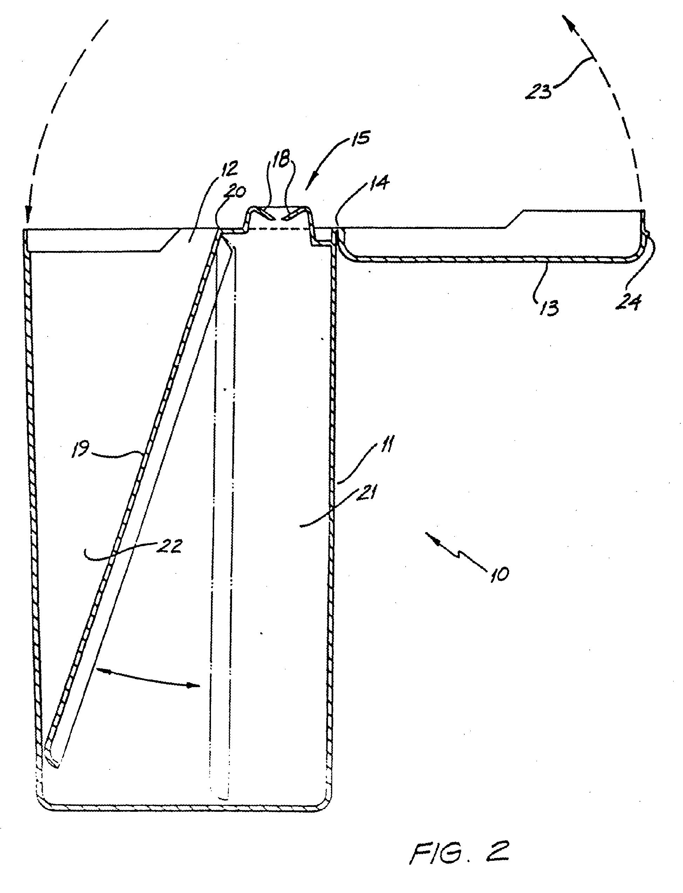

Figure 2 is a sectional elevational view through section line 2-2 of Figure 1,
Figure 3 is a plan view of an embodiment of the present invention showing the lid in the fully opened position, and
Figure 4 is a sectional elevational view through section line 4-4 of Figure 3.

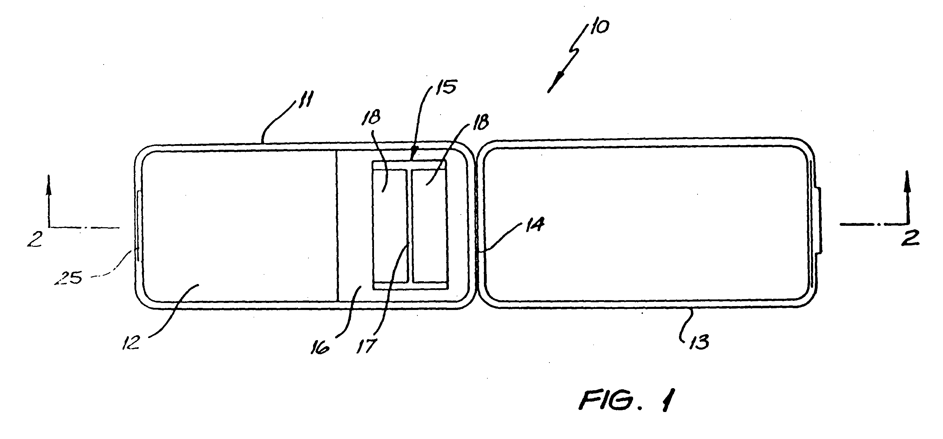

As illustrated in Figure 1, which is not an illustration of the claimed invention, a container 10 is provided comprising a substantially hollow shell 11 having an open end 12, a lid 13 which is mounted to the shell 11 by hinge 14 and retaining means 15 arranged to retain soiled syringes in the container.

The retaining means 15 is in the form of a frame 16 defining a rectangular aperture 17 which is substantially closed by two resiliently deformable rectangular lugs 18 extending into the aperture. Alternatively, the aperture could be of any desired shape and could for example be circular in which case there would be a number of substantially triangularly shaped lugs which substantially close the aperture. A dividing plate 19 is mounted to the frame by hinge 20 and is biased to the position illustrated by the full lines. The dividing plate 19 divides the body of the container into a soiled syringe retaining area 21 and a sterile syringe containing area 22.

In use, sterile syringes in their plastic wrapping are packed into area 22 and in so doing move dividing plate 19 to the position illustrated by the dashed lines. Sterile syringes are then removed from area 22, used and subsequently returned to the container by passing them through aperture 17 between lugs 18. Following passage through the aperture 17, a soiled syringe is retained in area 21 and as this process is repeated the dividing plate 19 progressively moves from right to left until eventually it adopts the position shown in the full lines with area 22 empty and area 21 full.

The container 10 can be closed by moving lid 13 about hinge 14 in the direction of arrow 23 with lug 24 ultimately engaging with recess 25 to snap fit the container closed and hide the contents from view.

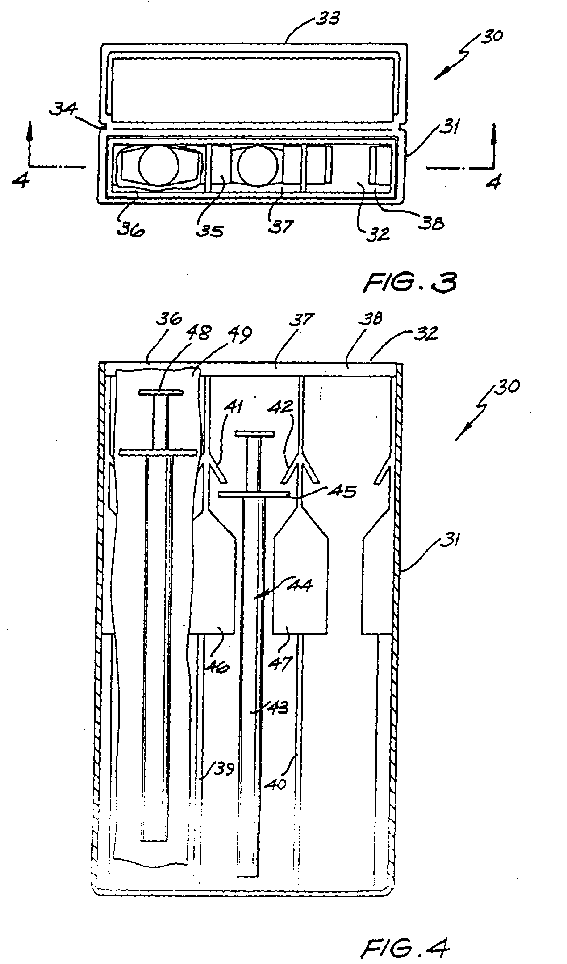

In an arrangement in accordance with the claimed invention, as illustrated in Figure 3, a container 30 is provided comprising a substantially hollow shell 31, having an open end 32, a lid 33 which is mounted to the shell 31, by hinge 34 and retaining means 35 arranged to retain soiled syringes in the container.

The retaining means 35 incorporate a number of apertures in the form of three similar pockets 36, 37 and 38. pocket 37 has two parallel walls 39 and 40 which extend away from the open end 32 of the shell. Resiliently deformable lugs 41 and 42 extend from walls 39 and 40 respectively and are biased to positions in which the body 43 of syringe 44 can pass between them with passage of flange 45 being restricted. Guiding protrusions 46 and 47 extend from walls 39 and 40 respectively to keep the body 43 of the syringe substantially parallel with the walls when passing between lugs 41 and 42.

In use, sterile syringe 48 housed within plastic wrapping 49 is fully contained within the container sitting in pocket 36, the flange of syringe 48 inhibiting the syringe from passing the lugs of pocket 36. Syringe 48 is therefore readily removed from the container, unwrapped and used and subsequently returned to the pocket which for purposes of illustration is to be considered pocket 37. The soiled syringe is inserted into the pocket and its travel is guided by protrusions 46 and 47 until the flange rests upon lugs 41 and 42. At that point, force is exerted down upon the syringe which deforms lugs 41 and 42 allowing the flange of the syringe to pass and thus retaining the syringe in the pocket whereby the syringe cannot be removed from the container without the application of excessive force to or destruction of the container.

The container 30 can be closed by moving lid 33 about hinge 34 until the lid force fits into the shell to close the container and hide the contents from view.

## Claims

1. A syringe container (30) for syringes of the type including a syringe body having a flanged section with generally radially outward projecting flange means thereon, the container comprising an open ended substantially hollow shell housing (31); retaining means (35) arranged to retain soiled syringes, and containing means arranged to contain sterile syringes **characterised in that** the retaining means (35) has two substantially parallel walls (39,40) extending away from the open end of the shell housing (31) and resiliently deformable lugs (41,42) which extend inwardly from each wall and are diametrically opposed and biased to positions in which the lugs are separated by a distance sufficient to permit passage of the body (43) of a syringe (44) but insufficient to permit passage of the flanged means (45) of the syringe (44) whereby the lugs (41,42) deform to permit passage of the flange means (45) upon application of sufficient force to the syringe (44).

2. A syringe container as claimed in claim 1, **characterised in that** the retaining means (35) is located within the containing means.

3. A syringe container as claimed in either of claims 1 or 2, **characterised in that** the retaining means (35) further comprises guide means (46,47) therein arranged to maintain the body (43) of the syringe (44) substantially parallel with the walls (39,40) thereof during passage of the syringe (44) between the lugs (41,42).

4. A syringe container as claimed in claim 3, **characterised in that** the guide means (46,47) comprise integrally moulded protrusions which extend inwardly from the walls (39,40), the protrusions (46,47) being located remote from the open end of the shell housing (31) and separated by a distance sufficient to permit passage of the syringe body (43) but insufficient to permit passage of the flange means (45).

5. A syringe container as claimed in any one of claims 2 to 4,
**characterised in that** the lugs (41,42) are located a distance from the open end of the shell housing (31) sufficient to allow a sterile syringe (44) to be completely contained within the container when the flange means (45) of the syringe is positioned on the top surfaces of the lugs (41,42).

6. A syringe container as claimed in any one of the preceding claims, **characterised in that** there are a plurality of retaining means (35).

7. A syringe container as claimed in any one of the preceding claims, **characterised in that** the shell housing (31) and the retaining means (35) are separately moulded from plastics material and ultra-sonically welded or otherwise fixed together.

8. A syringe container as claimed in any one of the preceding claims, including a closure lid (33) which is adapted to close said open end of said housing (31).
